# FASCICULE DE BREVET EUROPEEN

(11) **EP 1 926 557 B1**
(45) Date de publication et mention de la délivrance du brevet: **24.03.2010**
(21) Numéro de dépôt: 06831202.4
(22) Date de dépôt: 20.09.2006
(51) Int. Cl.: B01L 3/14, C12M 3/00

(54) **KIT POUR PREPARER UNE COMPOSITION COMPRENANT DES CELLULES ADIPOCYTAIRES**
AUSRÜSTUNG ZUR HERSTELLUNG EINER FETTZELLENHALTIGEN ZUSAMMENSETZUNG
KIT FOR PREPARING A COMPOSITION COMPRISING FAT CELLS

(30) Priorité: 20.09.2005 FR 0552827
(43) Date de publication de la demande: 04.06.2008
(73) Titulaire: Université de la Réunion, 97490 Saint Denis de la Réunion (FR); Roche, Régis, 97417 La Montagne de la Réunion (FR); Festy, Franck, 97419 La Possession de la Reunion (FR)
(72) Inventeur: ROCHE, Régis, 97417 La Montagne de La Reunion (FR); FESTY, Franck, Ruisseau Blanc 97419 La Montagne de La Reunion (FR)
(74) Mandataire: Michelet, Alain
(86) Numéro de dépôt international: PCT/FR2006/050913
(87) Numéro de publication internationale: WO 2007/034115

(56) Documents cités:
- WO-A-2006/013599
- DE-A1- 2 048 394
- US-A- 4 198 974
- US-A- 4 236 647
- US-A- 4 358 425
- US-A- 4 364 903
- US-A- 5 779 074
- US-A- 5 952 215
- US-A1- 2002 132 369
- US-A1- 2003 082 152
- US-A1- 2004 106 196
- US-B1- 6 382 441

## Description

La présente invention se rapporte au domaine de l'obtention de cellules adipocytaires, et à l'application des compositions cellulaires résultantes pour la fabrication de compositions à visée esthétique ou médicale pour régénérer localement les tissus, y compris les tissus adipeux.

De nombreux procédés d'obtention de cellules précurseurs d'adipocytes sont connus dans l'état de la technique.

En général, de tels procédés ont en commun une étape d'obtention d'un tissu adipeux de départ, provenant d'un lipoaspirat, qui est suivie d'une étape de digestion enzymatique du tissu adipeux, afin de libérer les cellules adipocytaires du tissu conjonctif environnant, puis une étape de purification des cellules précurseurs d'adipocytes.

En général, les cellules précurseurs d'adipocytes sont ensuite utilisées pour leur capacité, en tant que cellules souches, à se différencier dans de nombreux types cellulaires distincts, utilisables en thérapie.

Un tel procédé est par exemple décrit dans la demande de brevet américain N°US 2003/0082152 publiée le 1er mai 2003. Selon cette demande de brevet américain, les cellules précurseurs d'adipocytes, une fois purifiées, sont mises en culture pendant des temps variables, et en présence de différents facteurs solubles, afin de se différencier en des cellules de divers types cellulaires. Par exemple, on induit la différenciation des cellules précurseurs d'adipocytes en tissu ostéogène lorsqu'elles sont incubées en présence d'une combinaison de dexaméthasone, de phosphate d'acide ascorbique, et de béta-glycérophosphate. Pour obtenir une différenciation des cellules précurseurs d'adipocytes en cellules de tissu chondrogénique, les cellules précurseurs sont incubées en présence de sérum de veau foetal, de TGF-béta 1 et d'insuline. Pour induire la différenciation des cellules précurseurs d'adipocytes en cellules de tissu myogénique, les cellules précurseurs d'adipocytes purifiées sont incubées en présence de sérum de cheval et d'hydrocortisone. Pour induire la différenciation des cellules précurseurs d'adipocytes en tissu adipogénique, les cellules précurseurs d'adipocytes purifiées sont incubées en présence d'une combinaison d'isobutyl-méthylxanthine, de dexaméthasone, d'insuline et d'indométacine.

Selon d'autres procédés, les cellules précurseurs d'adipoçytes, après avoir subi une étape de purification, sont administrées aux patients sans la mise en oeuvre d'une étape de différenciation préalable.

De tels procédés d'obtention de cellules précurseurs d'adipocytes comprennent une étape de purification des cellules précurseurs d'adipocytes qui peut être d'une durée et d'une complexité variable. L'étape de purification peut consister, soit en une étape de sélection positive des cellules précurseurs d'adipocytes, soit en une étape de sélection négative dans laquelle les cellules non désirées sont éliminées, ou encore en une combinaison d'une étape de sélection positive et d'une étape de sélection négative. La mise en oeuvre d'étapes de sélection positive et/ou d'étapes de sélection négative dans un procédé de préparation d'une fraction cellulaire enrichie en précurseurs d'adipocytes est par exemple décrite dans la demande de brevet américain N°US 2004/0106196 publiée le 3 juin 2004.

Dans la demande de brevet N°US 2004/0106196, on décrit par exemple une étape de sélection positive des cellules précurseurs d'adipocytes par adhérence sélective des cellules précurseurs, par exemple sur certains types de supports pour membrane de filtration. On peut encore faire usage d'anticorps, ou de combinaison d'anticorps reconnaissant les molécules de surface exprimées sur les cellules précurseurs, ou au contraire sur les autres cellules de la population cellulaire de départ. De tels anticorps sont immobilisés sur un support solide, afin de retenir sélectivement les cellules qui expriment à leur surface les antigènes à l'encontre desquels les anticorps immobilisés sont dirigés. La purification des cellules précurseurs d'adipocytes peut également être réalisée par séparation immuno-magnétique à l'aide d'anticorps appropriés. Egalement, l'étape de purification peut être réalisée par une première étape d'incubation des cellules avec des anticorps dirigés contre des antigènes de surface cellulaire, puis passage des cellules ainsi traitées sur une colonne d'immuno-chromatographie sur laquelle sont immobilisés les anticorps secondaires se fixant sur les anticorps frimaires précédemment dtés. L'étape de purification cellulaire peut également être réalisée par la mise en oeuvre de gradients de densité, continus ou discontinus. L'étape de purification des précurseurs d'adipocytes peut également être réalisée à l'aide d'appareils de tri cellulaire, tels que des appareils d'élutriation, avec ou sans contre-courant L'état de purification des précurseurs d'adipocytes peut également être réalisé grâce à une étape d'adhésion sélective de la population cellulaire de départ sur la surface plastique d'un support de culture.

Après purification, les cellules précurseurs d'adipocytes purifiées comme décrit ci-dessus peuvent être directement administrées chez les patients.

On a aussi décrit un procédé d'obtention d'une fraction enrichie en cellules adipocytaires comprenant une étape de désagrégation de fragments de tissu adipeux d'un lipoaspirat dans un premier conteneur, puis une étape d'enrichissement cellulaire dans un second conteneur, par exemple par séparation sur gradient de densité, centrifugation, élutriation, adhérence cellulaire différentielle, sélection par des anticorps ou encore à l'aide de billes immuno-magnétiques (voir demande de brevet aux Etats-Unis n° US 2004/0106196).

Selon les objectifs qui sont poursuivis; les procédés de l'état de la technique décrits ci-dessus peuvent se révéler pleinement satisfaisants, notamment pour préparer des populations de cellules différenciées vers différents types tissulaires, ou encore lorsque les applications thérapeutiques envisagées nécessitent un très haut degré de pureté des cellules précurseurs d'adipocytes.

Toutefois, les procédés d'obtention de cellules précurseurs d'adipocytes décrits dans l'état de la technique, et dont les principaux d'entre eux sont illustrés ci-dessus, sont longs, et coûteux et sont donc destinés à corriger des situations physiologiques, notamment pathologiques, susceptibles de mettre en jeu la vie du patient.

Or, il existe un besoin dans l'état de la technique pour des procédés d'obtention de cellules précurseurs adipocytaires qui soient plus simples, plus rapides à réaliser et beaucoup moins coûteux que les procédés connus.

Selon l'invention, il a été mis au point un procédé d'obtention d'une fraction cellulaire enrichie en cellules adipocytaires, y compris d'une fraction enrichie en cellules précurseurs adipocytaires et d'une fraction enrichie en cellules d'adipocytes matures, plus simple et moins coûteux que les procédés connus, ainsi qu'un kit spécifiquement conçu pour la mise en oeuvre de ce nouveau procédé.

Les « cellules adipocytaires » selon l'invention englobent les cellules précurseurs et les cellules d'adipocytes mâtures.

Par « cellules précurseurs » ou « cellules précurseurs adipocytaires », on entend selon l'invention les cellules souches contenues dans le tissu adipeux et qui sont capables, dans les conditions appropriées, de se différencier en des cellules de divers types cellulaires, comme cela a été décrit dans l'état de la technique.

Selon l'invention, les « cellules précurseurs » englobent les cellules multipotentes et pluripotentes qui sont capables de se différencier en une diversité de types cellulaires, y compris les cellules adipogéniques, chrondrogéniques, cardiogéniques, dermatogéniques, hématopoïétiques, hémangiogéniques, myogéniques, néphrogéniques, urogénitogéniques, ostéogéniques, péricardiogéniques ou encore stromales.

Le demandeur s'est attaché à mettre au point un kit destiné à la préparation d'une composition comprenant des cellules adipocytaires qui permet la mise en oeuvre d'un procédé simple d'obtention d'une telle composition, utilisable en routine par le praticien et qui ne nécessite pas d'utiliser des appareillages coûteux.

Le demandeur a ainsi recherché la mise au point d'un kit pour préparer une composition comprenant des cellules adipocytaires purifiées qui permette au praticien d'utiliser la composition finale comprenant des cellules adipocytaires purifiées, le cas échéant de manière extemporanée, chez l'homme ou l'animal.

Au cours de cette recherche, le demandeur a mis au point un tube stérile, apte à être centrifugé, et qui puisse être utilisé de façon répétée dans des conditions stériles, et si possible également dans des conditions apyrogènes, au cours d'un procédé d'obtention d'une composition comprenant des cellules adipocytaires, qui peut être utilisée extemporanément chez l'homme ou l'animal.

Ainsi, l'invention a pour objet un tube stérile (1) apte à être centrifugé, comprenant un corps (11) et un bouchon (12) comprenant un élément en matériau élastique et rétractable, le corps (11) étant hermétiquement fermé par le bouchon (12), ledit bouchon (12) comprenant un orifice (13) permettant les échanges gazeux entre l'intérieur du tube et le milieu extérieur, ledit orifice (13) étant muni d'une membrane de filtre ayant une taille de pore inférieure à 0,30 µm.

Les différentes caractéristiques additionnelles susceptibles de caractériser le tube (1) ci-dessus sont décrites ci-dessous en relation avec les caractéristiques du kit dans lequel le tube (1) peut être inclus.

L'invention a aussi pour objet un kit pour préparer une composition comprenant des cellules précurseurs adipocytaires purifiées pour une utilisation extemporanée chez l'homme ou l'animal, ledit kit comprenant :
a) au moins un tube stérile (1) apte à être centrifugé, comprenant un corps (11) et un bouchon (12) comprenant un élément en matériau élastique et rétractable, le corps (11) étant hermétiquement fermé par le bouchon (12), ledit bouchon (12) comportant un orifice de sortie d'air (13) muni d'une membrane de filtre ayant une taille de pore inférieure à 0,30 µm ;
b) au moins une aiguille stérile comprenant un canal central, dont l'une des deux extrémités est munie d'un dispositif de fixation à une seringue, et dont l'autre extrémité est en forme de biseau, le diamètre du canal central étant de 3 millimètres ou plus, préférentiellement de 5 millimètres ou plus.

Grâce au kit ci-dessus, l'homme du métier peut obtenir, la plupart du temps moins de deux heures après l'obtention d'un lipoaspirat ou d'une lipectomie prélevé chez un patient, une composition comprenant des cellules précurseurs adipocytaires purifiées qui est directement utilisable pour son injection au même patient, en particulier, à des fins de reconstruction corporelle ou à des fins thérapeutiques.

Par « lipoaspirat », on entend selon l'invention un échantillon de tissu obtenu par une opération de liposuccion.

Par « lipectomie » on entend selon l'invention un échantillon de tissu, préférentiellement un échantillon de tissu adipeux, ledit échantillon de tissu contenant des adipocytes, ledit échantillon de tissu étant prélevé chez un patient par un acte chirurgical.

Le kit ci-dessus est particulièrement adapté pour la mise en oeuvre d'un procédé d'obtention d'une composition comprenant des cellules adipocytaires purifiées comprenant les étapes suivantes :
a) introduire une suspension de tissu adipeux provenant d'un lipoaspirat dans un tube (1) tel que défini ci-dessus. ;
b) réaliser, dans le tube (1), une digestion enzymatique de la suspension de tissu adipeux à l'aide d'une préparation enzymatique contenant au moins une protéase ;
c) centrifuger la suspension après digestion enzymatique ;
d) collecter les fractions de la suspension centrifugée contenant les cellules d'intérêt :
   d1) collecter la fraction (PA) de cellules précurseurs adipocytaires, qui est localisée en culot au fond du tube (1) ;
   d2) le cas échéant, collecter la fraction de cellules adipocytes matures (AM) retrouvée dans la partie supérieure de la suspension de tissu adipeux centrifugés à l'étape c) ;
e) incuber la fraction (PA) dans une solution hypotonique, afin de réaliser une lyse des hématies ; et
f) réaliser une composition de cellules adipocytaires à partir de l'une des fractions cellulaires f1), f2) ou f3) suivantes :
   f1) la fraction (PA) de cellules précurseurs adipocytaires telle qu'obtenue à la fin de l'étape e) ;
   f2) la fraction (AM) de cellules adipocytaires matures telle qu'obtenue à l'étape d1) ; et
   f3) un mélange des cellules de la fraction (PA) et des cellules de la fraction (AM).

Les compositions obtenues avec le procédé de l'invention peuvent être utilisées de manière extemporanée chez l'homme ou l'animal.

En particulier, le kit, selon l'invention, est particulièrement adapté pour la mise en oeuvre de procédés d'obtention de fractions enrichies en cellules précurseurs qui ne comprennent aucune étape complexe de purification à haut niveau des cellules précurseurs adipocytaires, autres que les étapes c), d) et e) du procédé général décrit ci-dessus.

Comme cela a déjà été mentionné, le kit de l'invention comprend au moins un tube stérile (1) tel que défini ci-dessus, qui est stérile et prêt à l'emploi pour réaliser les étapes a) à d), ou même les étapes a) à e) du procédé ci-dessus.

Par exemple, le tube (1) peut être utilisé jusqu'à l'étape e), ou même dans certains cas jusqu'à l'étape f), du procédé général défini ci-dessus lorsque, après centrifugation de la suspension traitée par la préparation enzymatique, la presque totalité de la suspension de tissu adipeux traitée par les enzymes et centrifugée est éliminée tout en conservant le culot cellulaire localisé au fond du tube (1) qui contient les cellules précurseurs, ledit culot cellulaire étant remis en suspension à l'étape e) avec la solution hypotonique, préalablement à la réalisation d'une ou plusieurs étapes de centrifugation et de lavage avec un milieu isotonique, puis la réalisation du mélange des cellules précurseurs avec le matériau de matrice biologique, à l'étape f) du procédé.

Afin de permettre la réalisation des étapes a) à d), ou alternativement des étapes a) à e), du procédé ci-dessus avec le seul tube stérile (1), ledit tube stérile (1) comprend un bouchon (12) comprenant un élément en matériau élastique et rétractable, afin que ledit bouchon (12) puisse être transpercé de multiples fois par l'aiguille d'une seringue, tout en conservant son étanchéité vis-à-vis de l'environnement extérieur après retrait de l'aiguille.

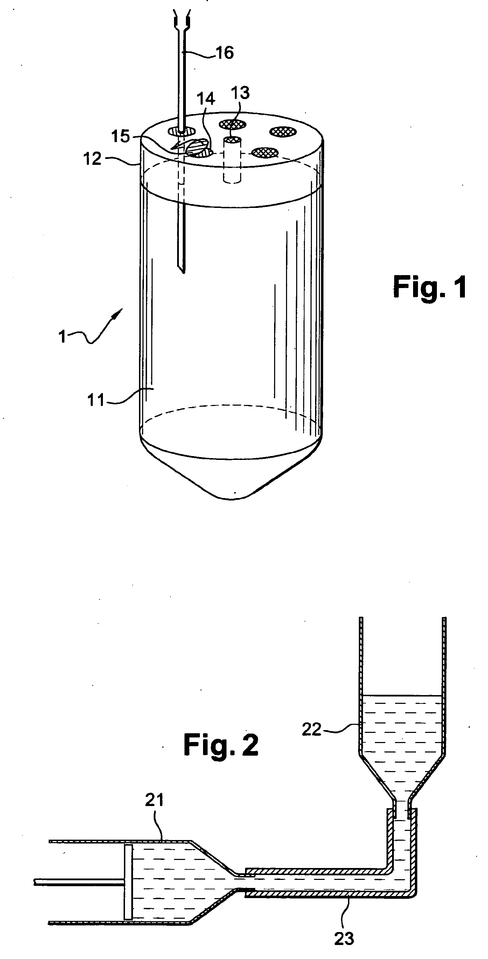

Selon une première alternative, la totalité du bouchon (12) est constituée en un matériau élastique et rétractable, comme cela est représenté sur la figure 1.

Selon une seconde alternative, seule une partie du bouchon (12) est réalisée en un matériau élastique et rétractable, de préférence la partie centrale du bouchon (12). Selon cette alternative, la partie centrale du bouchon (12) qui est constituée d'un matériau élastique et rétractable, est insérée, par exemple sertie ou enchâssée, dans la partie extérieure du bouchon (12). La partie extérieure du bouchon (12), qui peut être en métal ou en matière plastique, est en contact avec le bord supérieur du corps (11) du tube (1), et assure l'étanchéité du tube (1).

Le bouchon (12) peut être vissé sur le tube (1), grâce à des pas de vis complémentaires localisés respectivement sur le bouchon (12) et à l'extrémité supérieure du corps (11) du tube (1). Dans d'autres modes de réalisation, le bouchon (12) est engagé par forçage dans l'extrémité supérieure du corps (11) du tube (1).

Notamment, le matériau élastique et rétractable du bouchon (12) permet de transpercer ce bouchon avec l'aiguille d'une seringue, au moins aux étapes suivantes du procédé général ci-dessus :
- à l'étape a) d'introduction de la suspension de tissu adipeux dans le tube (1);
- le cas échéant, à l'étape b) lorsqu'une préparation enzymatique est introduite dans le tube (1), dans les modes de réalisation du procédé dans lesquels le tube (1) ne contient pas initialement une telle préparation enzymatique, sous forme liquide ou lyophilisée;
- à l'étape d) dans laquelle les différentes fractions cellulaires de la suspension centrifugée contenant les cellules d'intérêt sont collectées, le cas échéant par élimination du volume de la suspension cellulaire se situant dans le tube (1) au dessus du culot localisé au fond du tube contenant la fraction de cellules précurseurs (PA) ;
- à l'étape e) dans laquelle la solution hypotonique peut être ajoutée directement dans le tube (1) afin de remettre en suspension la fraction cellulaire contenant les cellules précurseurs;
- pendant la ou les étapes de lavage et centrifugation de la fraction cellulaire obtenue à la fin de l'étape e), avec un milieu isotonique, afin d'éliminer les hématies qui ont été lysées par l'incubation précédente avec le milieu hypotonique ; et
- le cas échéant, lors de l'étape f) de réalisation d'un mélange des cellules précurseurs adipocytaires avec le matériau de matrice biologique, étape au cours de laquelle le matériau de matrice biologique peut être ajouté directement dans le tube (1) qui contient la suspension cellulaire dans un milieu isotonique.

La figure 1 illustre un mode de réalisation particulier du tube (1) contenu dans le kit selon l'invention.

Dans ce mode de réalisation avantageux, le bouchon (12) du tube stérile (1) comprend au moins une surface de perforation (14) stérile. La stérilité de la surface de perforation (14) est préservée dans le temps grâce à une pellicule amovible (15) qui recouvre la surface de perforation (14). La pellicule amovible (15) est constituée en un matériau étanche aux liquides et éventuellement également étanche aux gaz. Par exemple, la pellicule amovible (15) peut consister en un film de paraffine ou encore en un film métallique, par exemple un film d'aluminium. Le cas échéant, la pellicule amovible (15) peut être retirée puis apposée de nouveau sur la surface de perforation (14) correspondante une pluralité de fois, afin de permettre une utilisation répétée de la surface de perforation (14) pour le passage de l'aiguille d'une seringue dans les différentes étapes d'addition ou de prélèvement de matériel dans ou à partir du tube (1), dès lors que les conditions de stérilité au moment de l'ajout ou du prélèvement de matériel dans ou à partir du tube (1) sont rigoureusement respectées.

Toutefois, de manière avantageuse, le bouchon (12) du tube stérile (1) comprend de multiples surfaces de perforation (14), en particulier au moins deux surfaces de perforation (14), par exemple de deux à cinq surfaces de perforation (14), afin de mettre en oeuvre le procédé général défini ci-dessus en utilisant qu'une seule fois une même surface de perforation (14) pour l'ajout ou le prélèvement de matériel dans ou à partir du tube (1).

Dans un autre mode de réalisation du bouchon (12) du tube stérile (1), celui-ci comprend plus de cinq surfaces de perforation (14). Ainsi, dans certains modes de réalisation du kit selon l'invention, le bouchon (12) du tube stérile (1) comprend jusqu'à dix surfaces de perforation (14).

En général, le tube (1) consiste en un tube dont le corps (1) est en matière plastique, par exemple en polypropylène, en polystyrène ou polyéthylène, d'un type connu et adapté pour la centrifugation de suspension cellulaire. Le tube (1) peut être d'un volume de 50 millilitres à 100 millilitres. Le tube (1) est adapté à une étape de centrifugation à une accélération au moins égale à 1000 g, de préférence au moins égale à 1500 g.

De manière générale, le tube (1) est un tube stérile et apyrogène.

Comme cela a déjà été mentionné, le bouchon (12) est constitué, au moins en partie, en un matériau élastique et rétractable de tout type connu par l'homme du métier, comme par exemple en un matériau de caoutchouc ou de silicone.

Selon une autre caractéristique, le bouchon (12) comprend un orifice de sortie d'air (13) muni d'une membrane de filtre ayant une taille ' de pore inférieure à 0,30 µm. L'orifice de sortie d'air (13) permet l'échange gazeux entre l'intérieur du tube (1) et le milieu extérieur, tout en empêchant le passage de particules, y compris de microorganismes, vers l'intérieur du tube (1). L'orifice de sortie d'air (13) permet d'ajouter ou de prélever facilement du matériel dans ou à partir du tube (1), sans simultanément créer de vide ou de surpression à l'intérieur du tube (1).

La membrane de filtre est d'un type connu de l'homme du métier, par exemple un filtre de nitrocellulose. La membrane du filtre qui équipe l'orifice de sortie d'air (13) peut posséder une taille de pore d'environ 0,2 µm, par exemple 0,22 µm.

Dans certains modes de réalisation du kit selon l'invention, le tube stérile (1) contient une préparation enzymatique apte à la digestion du tissu adipeux, ladite préparation enzymatique pouvant être sous forme liquide ou sous forme solide, par exemple, sous la forme d'une préparation enzymatique lyophilisée. De manière tout à fait préférée, ladite préparation enzymatique est stérile et apyrogène. Selon ce mode de réalisation du kit de l'invention, l'étape b) du procédé général défini ci-dessus peut être réalisée immédiatement après l'introduction de la suspension de tissu adipeux dans le tube (1).

Dans d'autres modes de réalisation du kit selon l'invention, le tube stérile (1) ne comprend pas de préparation enzymatique. Dans cet autre mode de réalisation du kit selon l'invention, ledit kit peut comprendre de plus, un conteneur, par exemple un tube ou un flacon, contenant une préparation enzymatique apte à la digestion du tissu adipeux, ladite préparation enzymatique étant sous forme liquide ou sous forme lyophilisée.

Le conteneur consiste en un conteneur stérile et apyrogène, hermétiquement bouché. Il peut être un flacon en matière plastique ou en verre, ou encore une ampoule de verre, ou encore une poche souple contentant la préparation enzymatique, de préférence sous forme liquide.

Avantageusement, la préparation enzymatique comprend au moins une protéase, et de préférence au moins une collagénase de tout type connu par l'homme du métier. A titre illustratif, l'homme du métier peut utiliser une préparation enzymatique décrite dans le brevet américain N°US 5, 952, 215 ou dans le brevet américain N°US 6, 475, 764.

Comme décrit ci-dessus, le kit selon l'invention comprend également au moins une aiguille stérile biseautée, adaptable à l'orifice de sortie d'une seringue et dont le diamètre du canal central est de 3 millimètres ou plus, préférentiellement de 5 millimètres ou plus. Cette aiguille stérile et apyrogène est utilisée à l'étape a) du procédé général ci-dessus pour introduire la suspension de tissu adipeux préalablement prélevée de manière stérile chez le patient dans le tube (1).

Avantageusement, cette aiguille d'un grand diamètre possède une extrémité biseautée afin d'éviter tout effet d'emporte-pièce au moment du transpercement du bouchon (12) du tube (1) au moment de son utilisation, à l'étape a) du procédé général ci-dessus. Cette aiguille stérile et apyrogène de grand diamètre doit être d'une longueur suffisante pour traverser le bouchon du tube. Avantageusement, cette aiguille stérile et apyrogène a une longueur supérieure ou égale à 50 millimètres.

L'autre extrémité de l'aiguille stérile et apyrogène de grand diamètre est munie d'un dispositif de fixation à une seringue, qui peut être un dispositif de fixation de tout type connu de l'homme du métier. Avantageusement, ledit dispositif de fixation à une seringue consiste en un système de verrouillage du type « Luer-Lock® » bien connu de l'homme du métier.

Dans certains modes de réalisation du kit selon l'invention, ledit kit comprend plusieurs tubes stériles et apyrogènes (1). En effet, il arrive fréquemment que le volume de prélèvement du tissu adipeux excède largement le volume utile d'un seul tube stérile (1), ce qui nécessite l'emploi, à l'étape a) du procédé général ci-dessus, de plusieurs tubes stériles (1). Avantageusement, un kit selon l'invention comprend 2, 3, 4, 6, 7 ou 8 tubes stériles (1).

Dans certains modes de réalisation du kit selon l'invention, ledit kit comprend de plus au moins un flacon contenant un milieu hypotonique adapté à la lyse des hématies. Le flacon contenant un milieu hypotonique, qui peut être de tout type connu de l'homme du métier, est avantageusement utilisé pour la réalisation de l'étape e) du procédé général défini ci-dessus.

A titre illustratif, ledit milieu hypotonique consiste en une solution aqueuse comprenant (i) de l'hydrogénocarbonate de potassium (KHCO₃) à la concentration finale de 10 mM, (ii) du chlorure d'ammonium (NH₄Cl) à la concentration finale de 155 mM et (iii) du sel disodique de l'acide éthylène diamine tétraacétique (Na₂ EDTA) à la concentration finale de 1 mM. Avantageusement, ledit milieu hypotonique possède un pH allant de 7,2 à 7,6.

Dans d'autres modes de réalisation selon l'invention, ledit kit comprend aussi au moins un flacon contenant un milieu isotonique adapté pour la remise en suspension des cellules, par exemple lors de la ou des étapes de lavage et centrifugation qui peuvent réalisées après l'étape e) de lyse des hématies, et avant le mélange de la fraction purifiée de cellules précurseurs adipocytaires avec le matériau de matrice biologique.

Avantageusement, le flacon contenant le milieu hypotonique peut être une poche de plastique souple, par exemple une poche de plastique souple d'un volume de 250 mL de type Ringer, comprenant un bouchon scellé en silicone qui est perforable par une aiguille. Le bouchon, par exemple de silicone, doit pouvoir être perforé, de préférence une pluralité de fois, avec une aiguille d'un diamètre de 18 Gauges sans affecter l'étanchéité du flacon contenant la solution hypotonique stérile et apyrogène. Dans certains modes de réalisation, la poche plastique contenant la solution hypotonique est placée dans un emballage stérile individuel que l'on peut ouvrir aisément. Selon encore d'autres modes de réalisation du kit selon l'invention, ledit kit comprend de plus au moins un dispositif stérile et apyrogène de filtration muni d'une membrane filtrante ayant une taille de pore allant de 40 µm à 200 µm.

Le dispositif stérile de filtration est utilisé dans certains modes de réalisation du procédé général défini précédemment, dans lesquels la suspension cellulaire obtenue à l'issue des étapes de lavage et centrifugation suivant l'étape e) de ce procédé est introduite par aspiration dans le volume intérieur d'une seringue, puis filtrée après avoir disposé le dispositif de filtration à l'embouchure de sortie de la seringue, afin d'éliminer de la suspension cellulaire les débris, notamment de tissus conjonctifs, afin d'obtenir après filtration une suspension contenant exclusivement, ou presque exclusivement, les cellules d'intérêt.

Le dispositif de filtration du kit selon l'invention est de tout type connu. Avantageusement, la membrane de filtration ayant une taille de pore allant de 40 µm à 200 µm, consiste en une membrane de filtration de nylon qui est maintenue en position fixe à l'intérieur du dispositif de filtration. Le dispositif de filtration lui-même est d'un type connu, par exemple du type des dispositifs de filtration adaptables à des seringues qui sont couramment commercialisées par les sociétés MILLIPORE ou SARTORIUS. Avantageusement, le dispositif de filtration comporte un système d'adaptation à l'embout d'une seringue constituée d'un moyen déverrouillage du type « Luer-Lock®». Avantageusement, le kit selon l'invention comprend aussi une aiguille adaptable à l'autre extrémité du dispositif de filtration, le cas échéant munie d'un système de verrouillage de type « Luer-Lock® », de manière à introduire la suspension de cellules précurseurs d'adipocytes dans un tube receveur, avantageusement un tube (1) stérile.

Dans certains modes de réalisation du kit selon l'invention, le filtre et l'aiguille sont solidaires l'un de l'autre, l'aiguille étant directement soudée au dispositif de filtration.

De préférence, l'aiguille destinée à être utilisée en combinaison avec le dispositif de filtration consiste en une aiguille d'au moins 16 Gauges, et dont la taille d'aiguille est suffisante pour traverser le bouchon du tube (1), la longueur de l'aiguille étant de préférence supérieure ou égale à 50 millimètres.

Dans ce mode de réalisation particulier d'un kit selon l'invention, ledit kit peut comprendre un ensemble comprenant la combinaison d'un dispositif de filtration et d'une aiguille tel que défini ci-dessus en plusieurs exemplaires.

Avantageusement, un kit selon l'invention comprend 2, 3, 4, 5, 6, 7, 8 exemplaires d'un ensemble comprenant la combinaison d'un dispositif de filtration et d'une aiguille tel que défini ci-dessus.

De manière générale, pour la mise en oeuvre du procédé général défini ci-dessus, on utilise autant d'ensembles comprenant la combinaison d'un dispositif de filtration et d'une aiguille que de tubes (1) stériles utilisés à l'étape a) du procédé.

Dans encore d'autres modes de réalisation du kit de l'invention, ledit kit comprend également au moins une aiguille d'au plus 18 Gauges possédant de préférence une longueur d'au moins 125 millimètres. Ladite aiguille est adaptée pour la mise en oeuvre de l'étape d1) du procédé, pour récupérer le surnageant de centrifugation ne contenant pas la fraction (PA) enrichie en cellules précurseurs adipocytaires. Ladite aiguille est également adaptée pour éliminer le liquide localisé dans le tube (1) au-dessous de la bande cellulaire contenant les cellules adipocytaires matures préalablement à la récupération des cellules précurseurs qui sont sous la forme d'un culot cellulaire au fond du tube (1).

Dans encore d'autres modes de réalisation du kit selon l'invention, ledit kit comprend de plus un dispositif permettant l'obtention d'une composition finale de cellules adipocytaires avec un matériau matrice, pour la mise en oeuvre de l'étape f) du procédé général défini dans la présente description.

Ainsi, dans certains modes de réalisation du procédé de l'invention, la composition finale est préparée à l'étape f) en combinant un matériau matrice avec les cellules adipocytaires, c'est-à-dire avec les cellules (PA), les cellules (AM), ou un mélange de ces cellules.

Par « matériau matrice », on entend selon l'invention un matériau non cytotoxique qui est stérile et apyrogène, et qui favorise l'implantation des cellules adipocytaires obtenues par le procédé, dans l'organisme humain ou animal.

Un tel dispositif (2) est illustré sur la figure 2 :
(i) une première seringue (21) destinée à être emplie avec un volume approprié d'un matériau matrice ;
(ii) une seconde seringue (22) destinée à être emplie d'une suspension de cellules précurseurs adipocytaires dans un milieu isotonique ; et
(iii) une tubulure. (23) reliant l'orifice de sortie de chacune des seringues (21 ; 22) ladite tubulure (23) mettant les seringues (21) et (22) en communication fluidique.

Avantageusement, les seringues (21) et (22) consistent en des seringues en plastique stériles et apyrogènes d'un type connu. Avantageusement, les seringues (21) et (22) consistent en des seringues ayant un volume utile d'au moins 50 millilitres et pouvant aller jusqu'à 100 millilitres ou plus.

Avantageusement, la tubulure (23) est une tubulure souple ou rigide, stérile et apyrogène. La tubulure (23) peut être en matière plastique souple ou en silicone. Dans un mode de réalisation particulier du dispositif (2), la première seringue (21) est pré-remplie avec un volume approprié du matériau matrice choisi.

Selon une autre caractéristique additionnelle du kit selon l'invention, ledit kit peut également comprendre au moins un portoir en plastique adapté pour recevoir les tubes (1). Le portoir de tubes peut consister en un portoir de tout type connu de l'homme du métier. Notamment, ledit portoir de tube peut être d'une conception très simple, suffisante pour maintenir les tubes (1) en position verticale pendant les diverses manipulations nécessaires à la réalisation du procédé général défini précédemment dans la description. Avantageusement, ledit portoir doit avoir une taille suffisante pour pouvoir maintenir en position verticale jusqu'à 20 tubes (1). Ledit portoir peut être d'une conception très simple et d'un coût de fabrication très faible.

La présente invention a encore pour objet un procédé d'obtention d'une composition de cellules adipocytaires, pour une utilisation chez l'homme ou l'animal, comprenant les étapes suivantes :
a) introduire une suspension de tissu adipeux provenant d'un lipoaspirat ou d'une lipectomie dans un tube (1), tel que défini dans la présente description;
b) réaliser, dans le tube (1), une digestion enzymatique de la suspension de tissu adipeux à l'aide d'une préparation enzymatique contenant au moins une protéase ;
c) centrifuger la suspension après digestion enzymatique ;
d) collecter les fractions de la suspension centrifugée contenant les cellules d'intérêt :
   d1) collecter la fraction (PA) de cellules précurseurs localisées en culot au fond du tube (1) ;
   d2) le cas échéant, collecter la fraction de cellules adipocytes matures (AM) retrouvées dans la partie supérieure de la suspension de tissu adipeux centrifugée ;
e) incuber la fraction PA dans une solution hypotonique, afin de réaliser une lyse des hématies ; et
f) réaliser une composition d'adipocytes à partir des fractions cellulaires suivantes :
   f1) la fraction (PA) de cellules précurseurs telle qu'obtenue à la fin de l'étape e).
   f2) la fraction (AM) de cellules d'adipocytes matures telle qu'obtenue à l'étape d1) ; et
   f3) un mélange des cellules de la fraction (PA), et des cellules de la fraction (AM).

Préalablement à l'étape a) du procédé, le tissu adipeux est prélevé de manière stérile, par lipo-aspiration selon les techniques connues de l'homme du métier, soit par aspiration manuelle, soit encore par aspiration à l'aide d'une pompe appropriée.

Le tissu adipeux prélevé est recueilli dans une seringue stérile, par exemple dans une seringue stérile d'un type muni d'un système de verrouillage à vis de type « Luer-Lock® ». Après décantation, le sérum est rejeté de la seringue.

Pour la suite du procédé, l'homme du métier utilise le kit selon l'invention qui a été défini en détail précédemment dans la présente description.

### Etape a) du procédé

En utilisant le kit selon l'invention, l'aiguille stérile constituant l'élément b) du kit, est adaptée à la seringue contenant le tissu adipeux. Puis, le contenu de la seringue est introduit dans le tube (1).

Dans certains cas, préalablement à l'étape a) du procédé, plusieurs seringues contenant du tissu adipeux sont obtenues après lipoaspiration. A l'étape a) du procédé, le contenu de plusieurs seringues contenant le tissu adipeux peut être introduit dans un seul tube (1).

Le cas échéant, lorsque le volume de tissu adipeux excède le volume utile du tube (1), plusieurs tubes (1) contenus dans un kit selon l'invention peuvent être utilisés afin de recueillir le tissu adipeux.

### Etape b) du procédé

Lorsque l'on utilise le mode de réalisation du kit selon l'invention dans lequel le tube (1) est pré-rempli avec une préparation enzymatique apte à la digestion du tissu adipeux, l'étape b) de digestion enzymatique peut être directement réalisée.

En revanche, dans le mode de réalisation du kit de l'invention dans lequel le tube (1) ne contient pas de préparation enzymatique, l'étape b) de digestion enzymatique est précédée par l'introduction dans le tube (1) de la quantité appropriée de préparation enzymatique contenue dans le kit dans un conteneur séparé.

Avantageusement, à l'étape b) du procédé, la préparation enzymatique comprend de la collagénase qui est utilisée à une concentration finale dans le tube (1) variant de 0,05 à 5 unités de collagénase par millilitres de tissu adipeux contenu dans le tube (1), l'unité de collagénase étant l'unité d'activité PZ telle que définie par Wünsch. Selon Wünsch, une unité de collagénase catalyse l'hydrolyse d'1µmole de 4-phénylazobenzyloxycarbonyl-L-prolyl-L-leucyl-glycyl-L-prolyl-D-arginine par minute à 25°C et à pH 7,1.

Lorsque la préparation enzymatique apte à la digestion du tissu adipeux se présente sous forme de poudre, par exemple sous forme lyophilisée, dans un conteneur séparé du tube (1), l'homme du métier reconstitue une solution liquide de préparation enzymatique en ajoutant audit conteneur un volume approprié d'un milieu isotonique compatible avec l'utilisation chez l'homme, par exemple un milieu isotonique de type Ringer-Lactate, afin de remettre en solution les enzymes. Puis, un volume défini de la solution enzymatique est introduit par injection dans le ou les tubes (1) contenant le tissu adipeux.

Avantageusement, une fois que le mélange du tissu adipeux avec la préparation enzymatique a été réalisé dans le ou les tubes (1), on ajoute un milieu physiologique compatible avec une administration chez l'homme dans chacun des tubes (1), afin de les remplir.

Le ou les tubes (1) sont ensuite incubés à une température pouvant varier de 10°C à 60°C, mieux de 25°C à 45°C, encore mieux de 30°C à 40°C, par exemple 37°C, afin de réaliser l'étape b) de digestion enzymatique. Lors de l'étape b), les tubes (1) sont de préférence soumis à une agitation continue. En général, à l'étape b), la digestion enzymatique a une durée variant de 5 minutes à 2 heures, mieux de 10 minutes à 30 minutes.

De manière facultative, à la fin de l'étape b), la suspension obtenue après digestion enzymatique peut être filtrée à travers un dispositif de filtration, de préférence un dispositif de filtration d'un type décrit précédemment dans la présente description, possédant une membrane de filtration ayant une taille de pore comprise entre 40 µm et 200 µm. Cette étape de filtration facultative permet d'éliminer les débris et résidus de tissu adipeux résultant de la digestion enzymatique, afin d'obtenir, à la fin de l'étape b), une suspension liquide contenant essentiellement des cellules.

### Etape c) du procédé

A l'étape c) du procédé, la suspension cellulaire, le cas échéant débarrassée de la majorité des débris du tissu adipeux par filtration, est centrifugée à une accélération comprise entre 10 g et 5000 g, mieux entre 800 g et 3000 g, encore mieux entre 1000 g et 2000 g.

L'étape c) de centrifugation a une durée allant de 5 secondes à 30 minutes, selon la vitesse de centrifugation qui a été choisie. A titre illustratif, la centrifugation peut être réalisée à 1500 g pendant 10 minutes.

A l'issue de l'étape c) de centrifugation, la fraction cellulaire enrichie en cellules précurseurs, aussi désignée fraction (PA) dans la présente description, est retrouvée en culot au fond du tube (1).

A l'issue de l'étape c) de centrifugation, une fraction cellulaire enrichie en cellules d'adipocytes matures est retrouvée sous la forme d'une bande cellulaire localisée dans la partie supérieure de la suspension liquide contenue dans le tube (1).

### Etape d) du procédé

### Etape d1) du procédé

A l'étape d1) du procédé, la fraction (PA) enrichie en cellules précurseurs est récupérée par élimination du liquide surnageant localisé dans le tube (1) au-dessus du culot cellulaire. Avantageusement, le surnageant est éliminé du tube (1) par aspiration à l'aide d'une seringue munie d'une aiguille stérile dont la longueur est au moins égale à la hauteur totale du tube (1).

### Etape d2) du procédé (facultative).

Dans certains modes de réalisation du procédé de l'invention, on récupère, à l'étape d2), également la fraction de cellules adipocytes matures, aussi appelée fraction (AM) dans la présente description, qui sont retrouvées dans la partie supérieure de la suspension de tissu adipeux centrifugée.

La récupération de la bande cellulaire contenant les adipocytes matures peut être réalisée à l'aide d'une seringue, munie d'une aiguille stérile et apyrogène, par aspiration de la bande cellulaire d'intérêt dans la seringue.

En général, la fraction cellulaire (AM) d'adipocytes matures est ensuite introduite dans un tube (1) vide.

Dans la pratique, lorsque l'étape d2) est effectuée, cette étape est généralement réalisée avant l'étape d1).

### Etape e) du procédé

A la suite de l'étape d1), la fraction cellulaire (PA) de cellules précurseurs est remise en suspension dans un milieu hypotonique, stérile et apyrogène, qui est introduit dans le tube (1) contenant le culot cellulaire issu de l'étape d1), à l'aide d'une seringue munie d'une aiguille stérile et apyrogène.

Après l'introduction du volume approprié du milieu hypotonique, les tubes (1) contenant les cellules précurseurs dans le milieu hypotonique sont fortement agitées puis soumis à une centrifugation. Avantageusement, les tubes (1) sont centrifugés à une accélération comprise entre 100 g et 5000 g pendant une durée allant de 30 secondes à 30 minutes. Les conditions de la centrifugation réalisée à la fin de l'étape e) du procédé sont en général identiques aux conditions de la centrifugation réalisée à l'étape c) du procédé.

A l'issue de la centrifugation ci-dessus, les cellules précurseurs sont retrouvées en culot au fond des tubes (1). Le surnageant liquide est éliminé du tube par aspiration, grâce à une seringue munie d'une aiguille stérile et apyrogène.

Puis, le culot cellulaire de chacun des tubes (1) est remis en suspension dans un milieu isotonique compatible avec l'injection chez l'homme, comme par exemple, un milieu isotonique du type Ringer-Lactate. Le milieu isotonique est introduit dans les tubes (1) à l'aide d'une seringue stérile munie d'une aiguille stérile et apyrogène.

Le cas échéant, les suspensions cellulaires ainsi obtenues sont réunies dans un seul tube (1), si leur volume le permet.

De manière facultative, la fraction cellulaire ainsi remise en suspension peut être soumise à une étape de filtration à l'aide d'un dispositif de filtration d'un type décrit précédemment dans la présente description, possédant une membrane filtrante ayant une taille de pore allant de 40 µm à 200 µm, afin d'éliminer de la fraction cellulaire les éventuels résidus indésirables de tissu adipeux ou de tissu conjonctif présents au sein de la suspension cellulaire.

Dans les modes de réalisation du procédé de l'invention, dans lesquels l'étape d2) est réalisée, les fractions cellulaires contenant les cellules d'adipocytes matures sont soumises à une centrifugation du même type que celle décrite ci-dessus pour la fraction cellulaire issue de l'étape d1), puis remise en suspension dans un milieu isotonique compatible avec une injection chez l'homme, par exemple du type Ringer-Lactate.

De manière générale, que ce soit pour les cellules précurseurs issues de l'étape d1) ou pour les cellules d'adipocytes matures issues de l'étape d2), il est souhaitable de réaliser plusieurs étapes de centrifugation puis remise en suspension des fractions cellulaires dans un milieu isotonique (lavage) avant l'obtention des fractions cellulaires respectivement de cellules précurseurs (PA) ou d'adipocytes matures (AM) finales utilisables pour la mise en oeuvre de l'étape f) du procédé.

### Etape f) du procédé

A l'étape f) du procédé, on réalise la composition finale comprenant des cellules de la fraction (PA), des cellules de la fraction (AM) ou d'un mélange de cellules de fraction (PA) et de cellules de la fraction (AM), par exemple avec un rapport cellulaire (PA)/(AM) variant de 1/99 à 99/1, le cas échéant en combinaison avec un ou plusieurs principes actifs pharmaceutiques et/ou un ou plusieurs excipients pharmaceutiquement acceptables. Par exemple, on peut combiner les cellules adipocytaires, avec un matériau de matrice, afin d'obtenir une composition finale comprenant des cellules adipocytaires purifiées. Une telle composition peut être utilisée de manière extemporanée et administrée directement chez l'homme ou l'animal.

Le matériau de matrice peut consister en un matériau de matrice biologique, qui peut être choisi parmi tous les matériaux de matrice biologique compatibles avec une administration chez l'homme ou l'animal, d'un type connu de l'homme du métier. Avantageusement, le matériau de matrice biologique est choisi parmi les matériaux matrice résorbables, qui englobent le collagène, l'acide hyaluronique ou encore des hydrogels tels que des hydrogels acryliques pouvant comprendre aussi de l'acide hyaluronique.

A titre illustratif, un matériau matrice résorbable comprenant du collagène peut être choisi parmi les matériaux commercialisés sous les noms Artecoll®, Zyderm®, Zyplast® ou encore Autologen®.

A titre illustratif, un matériau matrice résorbable comprenant de l'acide hyaluronique peut être choisi parmi les matériaux commercialisés sous les noms Hylaform®, Restylane®, Perlane®, Juvederm®, Hylan® ou encore Hydrafill®.

A titre illustratif, un matériau matrice résorbable à base d'un hydrogel acrylique comprenant de l'acide hyaluronique peut être choisi parmi les matériaux commercialisés sous les noms Derlakuve® ou encore Dermadeep®.

Le matériau matrice peut aussi être choisi parmi les matériaux matrice non résorbables, qui englobent les matériaux à base de polymères naturels ou synthétiques tels que le polytétrafluoréthylène expansé, le polyester expansé, l'acide poly-L-lactique, les polyacrylamides réticulés ou encore le poly-alkyl-imide.

A titre illustratif un matériau matrice non résorbable à base de polytétrafluoroéthylène expansé peut être choisi parmi les matériaux commercialisés sous les noms Softform® ou encore Gortex®.

A titre illustratif un matériau matrice non résorbable à base de polyester expansé élastique peut être choisi parmi les matériaux commercialisés sous les noms M-SI Fil® ou encore Filladerm®.

A titre illustratif, un matériau matrice non résorbable à base d'acide poly-L-lactique, par exemple à base de microsphères d'acide poly-L-lactique, peut consister en un matériau commercialisé sous la dénomination New File®.

A titre illustratif, un matériau matrice non résorbable à base d'un polyacrylamide réticulé peut consister en un matériau commercialisé sous la dénomination Aquamid®.

A titre illustratif, un matériau matrice non résorbable à base de poly-alkyl-imide peut consister en un matériau commercialisé sous la dénomination Bio-alcamid®.

A titre illustratif, on peut utiliser, comme matériau de matrice biologique, l'acide hyaluronique réticulé ou non réticulé.

Avantageusement, à l'étape f) du procédé, la composition finale contenant les cellules précurseurs d'adipocytes prêtes à l'emploi pour l'administration chez l'homme comprend une quantité de matériau matrice 0,001 à 10 grammes dudit matériau pour un volume de 100 millilitres de ladite composition finale. De préférence, la composition finale prête à l'emploi comprend de 0,1 à 1 gramme de matériau matrice biologique, pour un volume de 100 millilitres de ladite composition finale.

A l'étape f) du procédé, on peut, dans certains modes de réalisation, inclure dans la composition finale de cellules adipocytaires un principe actif ou une combinaison de principes actifs pharmaceutiques à usage humain ou vétérinaire.

A titre illustratif, la composition finale de cellules adipocytaires peut comprendre un ou plusieurs facteurs de croissance, comme par exemple un facteur de croissance des fibroblastes (FGF), le facteur de croissance de l'épiderme (EGF) ou encore un facteur de stimulation des colonies (CSF).

A titre illustratif, une composition finale prête à l'emploi selon l'invention comprend de 0,2 à 0,5 grammes d'acide hyaluronique non réticulé pour 100 millilitres de composition finale.

De manière générale, la composition finale prête à l'emploi selon l'invention comprend de 10³ à 10⁹ cellules d'intérêt par millilitre de composition finale, mieux de 10⁴ à 10⁸ cellules d'intérêt par millilitre de composition finale et encore mieux de 5 à 10 x 10⁶ cellules d'intérêt par millilitre de composition finale.

En dernier lieu, l'invention a pour objet un tube (1) stérile tel qu'il est défini en détail dans la présente description.

## Revendications

1. Kit pour préparer une composition comprenant des cellules adipocytaires, ledit kit comprenant :
a) au moins un tube stérile (1) apte à être centrifugé, comprenant un corps (11) et un bouchon (12) comprenant un élément en matériau élastique et rétractable, le corps (11) étant hermétiquement fermé par le bouchon (12), ledit bouchon (12) comportant un orifice de sortie d'air (13) muni d'une membrane de filtre ayant une taille de pore inférieure à 0,30 µm ;
b) au moins une aiguille stérile comprenant un canal central, dont l'une des deux extrémités est munie d'un dispositif de fixation à une seringue, et dont l'autre extrémité est en forme de biseau, le diamètre du canal central étant de 3 millimètres ou plus, préférentiellement de 5 millimètres ou plus.

2. Kit selon la revendication 1, **caractérisé en ce que** le bouchon (12) du tube stérile (1) comprend au moins une surface de perforation (14) stérile recouverte d'une pellicule amovible (15).

3. Kit selon la revendication 2, **caractérisé en ce que** le bouchon (12) du tube stérile (1) comprend de 2 à 5 surfaces de perforation (14).

4. Kit selon la revendication 2, **caractérisé en ce que** le bouchon (12) du tube stérile (1) comprend plus de 5 surfaces de perforation (14).

5. Kit selon l'une des revendications 1 à 4, **caractérisé en ce que** le tube stérile (1) contient une préparation enzymatique apte à la digestion du tissu adipeux, ladite préparation enzymatique étant sous forme liquide ou sous forme lyophilisée.

6. Kit selon l'une des revendications 1 à 4, **caractérisé en ce que** le tube stérile (1) ne comprend pas une préparation enzymatique.

7. Kit selon la revendication 6, **caractérisé en ce qu'**il comprend un conteneur contenant une préparation enzymatique apte à la digestion du tissu adipeux, ladite préparation enzymatique étant sous forme liquide ou sous forme lyophilisée.

8. Kit selon l'une des revendications 1 à 7, **caractérisé en ce qu'**il comprend 2, 3, 4, 5, 6, 7 ou 8 tubes stériles (1).

9. Kit selon l'une des revendications 1 à 8, **caractérisé en ce qu'**il comprend un flacon contenant un milieu hypotonique adapté pour la lyse des hématies.

10. Kit selon l'une des revendications 7 ou 8, **caractérisé en ce que** ledit milieu hypotonique possède un pH allant de 7,2 à 7,6.

11. Kit selon l'une des revendications 1 à 10, **caractérisé en ce qu'**il comprend un flacon contenant un milieu isotonique adapté pour la remise en suspension des cellules.

12. Kit selon l'une des revendications 1 à 11, **caractérisé en ce qu'**il comprend au moins un dispositif stérile de filtration muni d'une membrane filtrante ayant une taille de pores allant de 40 µm à 200 µm.

13. Kit selon la revendication 12, **caractérisé en ce que** le dispositif stérile de fiitration comprend aussi une aiguille stérile d'au moins 16 Gauges.

14. Kit selon l'une des revendications 1 à 13, **caractérisé en ce qu'**il comprend aussi au moins une aiguille d'au plus 18 Gauges.

15. Kit selon l'une des revendications 1 à 14, **caractérisé en ce qu'**il comprend de plus un dispositif (2) permettant l'obtention d'une composition de mélange de cellules précurseurs avec une matrice biologique, ledit dispositif étant constitué de :
(i) une première seringue (21) destinée à être emplie avec un volume approprié d'une matrice biologique ;
(ii) une seconde seringue (22) destinée à être emplie d'une suspension de cellules précurseurs dans un milieu isotonique ; et
(iii) une tubulure (23) reliant l'orifice de sortie de chacune des seringues (21, 22) ladite tubulure (23) mettant les seringues (21) et (22) en communication fluidique.

16. Procédé d'obtention d'une composition contenant des cellules adipocytaires, comprenant les étapes suivantes :
a) introduire une suspension de tissu adipeux provenant d'un lipoaspirat ou d'une lipectomie dans un tube (1), tel que défini dans la revendication 1 ;
b) réaliser, dans le tube (1), une digestion enzymatique de la suspension de tissu adipeux à l'aide d'une préparation enzymatique contenant au moins une protéase ;
c) centrifuger la suspension après digestion enzymatique ;
d) collecter les fractions de la suspension centrifugée contenant les cellules d'intérêt :
d1) collecter la fraction (PA) de cellules précurseurs adipocytaires localisées en culot au fond du tube (1) ;
d2) le cas échéant, collecter la fraction de cellules adipocytes matures (AM) retrouvées dans la partie supérieure de la suspension de tissu adipeux centrifugée ;
e) incuber la fraction (PA) dans une solution hypotonique, afin de réaliser une lyse des hématies ; et
f) réaliser une composition de cellules adipocytaires à partir de l'une des fractions cellulaires suivantes :
f1) la fraction (PA) de cellules précurseurs adipocytaires précurseurs telle qu'obtenue à la fin de l'étape e).
f2) la fraction (AM) de cellules d'adipocytes matures telle qu'obtenue à l'étape d1) ; et
f3) un mélange des cellules de la fraction (PA), et des cellules de la fraction (AM).

17. Procédé selon la revendication 16, **caractérisé en ce qu'**à l'étape f), lorsque le mélange est réalisé à la fois avec les cellules de la fraction (PA) et avec les cellules de la fraction (AM), le rapport cellulaire (PA)/(AM) varie de 1/99 à 99/1.

18. Un tube stérile (1) apte à être centrifugé, comprenant un corps (11) et un bouchon (12) comprenant un élément en matériau élastique et rétractable, le corps (11) étant hermétiquement fermé par le bouchon (12), ledit bouchon (12) comportant un orifice de sortie d'air (13) muni d'une membrane de filtre ayant une taille de pore inférieure à 0,30 µm.

19. Tube stérile (1) selon la revendication 18, **caractérisé en ce que** le bouchon (12) du tube stérile (1) comprend au moins une surface de perforation (14) stérile recouverte d'une pellicule amovible (15).

20. Tube stérile (1) selon l'une des revendications 18 et 19, **caractérisé en ce que** le bouchon (12) du tube stérile (1) comprend au moins deux surfaces de perforation (14).

21. Tube stérile (1) selon l'une des revendications 18 à 20, **caractérisé en ce qu'**il contient une préparation enzymatique apte à la digestion du tissu adipeux, ladite préparation enzymatique étant sous forme liquide ou sous forme lyophilisée.

## Claims

1. A kit for preparing a composition comprising fat cells, said kit comprising:
a) at least one sterile tube (1) adapted to be centrifuged, including a body (11) and a plug (12) comprising an element made of elastic and retractable material, the body (11) being hermetically sealed by the plug (12), said plug (12) including an air outlet (13) provided with a filter membrane having a size of pores of less than 0.30 µm;
b) at least one sterile needle comprising a central channel, one end of which is provided with a device for being fixed to a syringe, and the other end of which is bevelled, the diameter of the central channel being of 3 millimeters or more, preferably of 5 millimeters or more.

2. A kit according to claim 1, **characterized in that** the plug (12) of the sterile tube (1) comprises at least one sterile puncture area (14) covered with a strippable film (15).

3. A kit according to claim 2, **characterized in that** the plug (12) of the sterile tube (1) comprises from 2 to 5 puncture areas (14).

4. A kit according to claim 2, **characterized in that** the plug (12) of the sterile tube (1) comprises more than 5 puncture areas (14).

5. A kit according to any of claims 1 to 4, **characterized in that** the sterile tube (1) contains an enzyme-based preparation suitable for adipose tissue digestion, said enzyme-based preparation being either in a liquid or in a lyophilized form.

6. A kit according to any of claims 1 to 4, **characterized in that** the sterile tube (1) does not comprise any enzyme-based preparation.

7. A kit according to claim 6, **characterized in that** it comprises a container containing an enzyme-based preparation suitable for adipose tissue digestion, said enzyme-based preparation being either in a liquid or in a lyophilized form.

8. A kit according to any of claims 1 to 7, **characterized in that** it includes 2, 3, 4, 5, 6, 7 or 8 sterile tubes (1).

9. A kit according to any of claims 1 to 8, **characterized in that** it comprises a flask containing hypotonic medium suitable for the erythrocyte lysis.

10. A kit according to claim 7 or 8, **characterized in that** said hypotonic medium has a pH value ranging from 7.2 to 7.6.

11. A kit according to any of claims 1 to 10, **characterized in that** it comprises a flask containing an isotonic medium suitable for cell resuspension.

12. A kit according to any of claims 1 to 11, **characterized in that** it comprises at least one sterile filtering device provided with a filter membrane having a pore size ranging from 40 µm to 200 µm.

13. A kit according to claim 12, **characterized in that** the sterile filtering device further comprises a sterile needle of not less than 16 Gauges.

14. A kit according to any of claims 1 to 13, **characterized in that** it further comprises at least one needle of not more than 18 Gauges.

15. A kit according to any of claims 1 to 14, **characterized in that** it further comprises a device (2) for obtaining a composition resulting from the combination of precursor cells with a biological matrix, said device being composed of:
(i) a first syringe (21) intended to be filled with a suitable volume of a biological matrix;
(ii) a second syringe (22) intended to be filled with a precursor cell suspension in an isotonic medium; and
(iii) a pipe (23) connecting the outlet of each syringe (21, 22), said pipe (23) putting the syringes (21) and (22) into fluid communication with each other.

16. A method for preparing a composition comprising fat cells, said method comprising following steps of:
a) introducing an adipose tissue suspension resulting from a liposuction or a lipectomy into a tube (1) such as defined in claim 1;
b) allowing an enzymatic digestion of the adipose tissue suspension to proceed in the tube (1) using an enzyme-based preparation containing at least one protease;
c) centrifuging the suspension after the enzymatic digestion;
d) collecting the centrifuged suspension fractions containing the interesting cells:
d1) collecting the adipocyte precursor cell (AP) fraction in the pellet located in the bottom portion of the tube (1);
d2) if necessary, collecting the mature adipocyte (MA) fraction recovered in the upper part of the centrifuged adipose tissue suspension;
e) incubating the (AP) fraction in a hypotonic solution so as to lyse erythrocytes; and
f) preparing a fat cell composition from one of the following cell fractions:
f1) the (AP) fraction of adipocyte precursor cells such as obtained at the end of step e);
f2) the (MA) fraction of mature adipocytes such as obtained in step d1); and
f3) a combination of cells of the (AP) fraction and cells of the (MA) fraction.

17. A method according to claim 16, **characterized in that** in step f), when the combination is made with both (AP) fraction cells and (MA) fraction cells, the cell ratio (AP):(MA) does vary from 1:99 to 99:1.

18. A sterile tube (1) adapted to be centrifuged, including a body (11) and a plug (12) comprising an element made of elastic and retractable material, the body (11) being hermetically sealed by the plug (12), said plug (12) including an air outlet (13) provided with a filter membrane having a size of pores of less than 0.30 µm.

19. A sterile tube (1) according to claim 18, **characterized in that** the plug (12) of the sterile tube (1) comprises at least one sterile puncture area (14) covered with a removable film (15).

20. A sterile tube (1) according to claim 18 or 19, **characterized in that** the plug (12) of the sterile tube (1) comprises at least two puncture areas (14).

21. A sterile tube (1) according to any of claims 18 to 20, **characterized in that** it contains an enzyme-based preparation suitable for adipose tissue digestion, said enzyme-based preparation being either in a liquid or in a lyophilized form.

## Patentansprüche

1. Set zur Herstellung einer Fettzellen enthaltenden Zusammensetzung, wobei das Set aufweist:
a) mindestens einem zentrifugierbaren sterilen Röhrchen (1) mit einem Körper (11) und einem Stopfen (12), der ein Element aus elastischem und zurückziehbarem Material aufweist, wobei der Körper (11) durch den Stopfen (12) hermetisch geschlossen ist, wobei der Stopfen (12) eine Luftauslassöffnung (13) aufweist, welche mit einer Filtermembran mit einer Porengröße von weniger als 0,30 µm versehen ist;
b) mindestens eine sterile Nadel mit einem mittigen Kanal, dessen eines seiner beiden Enden mit einer Vorrichtung zur Befestigung an einer Spritze versehen ist, und dessen anderes Ende als Fase ausgebildet ist, wobei der Durchmesser des mittigen Kanals 3 Millimeter oder mehr, vorzugsweise 5 Millimeter oder mehr beträgt.

2. Set nach Anspruch 1, **dadurch gekennzeichnet, dass** der Stopfen (12) des sterilen Röhrchens (1) mindestens eine sterile Perforationsfläche (14) aufweist, die mit einer lösbaren Folie (15) bedeckt ist.

3. Set nach Anspruch 2, **dadurch gekennzeichnet, dass** der Stopfen (12) des sterilen Röhrchens (1) 2 bis 5 Perforationsflächen (14) aufweist.

4. Set nach Anspruch 2, **dadurch gekennzeichnet, dass** der Stopfen (12) des sterilen Röhrchens (1) mehr als 5 Perforationsflächen (14) aufweist.

5. Set nach einem der Ansprüche 1 bis 4, **dadurch gekennzeichnet, dass** das sterile Röhrchen (1) eine für die Verdauung des Fettgewebes geeignete enzymatische Zubereitung enthält, wobei die enzymatische Zubereitung in flüssiger oder lyophilisierter Form vorliegt.

6. Set nach einem der Ansprüche 1 bis 4, **dadurch gekennzeichnet, dass** das sterile Röhrchen (1) keine enzymatische Zubereitung enthält.

7. Set nach Anspruch 6, **dadurch gekennzeichnet, dass** es einen Behälter aufweist, der eine zur Verdauung des Fettgewebes geeignete enzymatische Zubereitung enthält, wobei die enzymatische Zubereitung in flüssiger oder lyophilisierter Form vorliegt.

8. Set nach einem der Ansprüche 1 bis 7, **dadurch gekennzeichnet, dass** es 2, 3, 4, 5, 6, 7 oder 8 sterile Röhrchen (1) aufweist.

9. Set nach einem der Ansprüche 1 bis 8, **dadurch gekennzeichnet, dass** es eine Flasche aufweist, welche ein für die Lyse von Erythrozyten geeignetes hypotonisches Medium enthält.

10. Set nach einem der Ansprüche 7 oder 8, **dadurch gekennzeichnet, dass** das hypotonische Medium einen pH-Wert von 7,2 bis 7,6 aufweist.

11. Set nach einem der Ansprüche 1 bis 10, **dadurch gekennzeichnet, dass** es eine Flasche aufweist, die ein isotonisches Medium enthält, um die Suspension der Zellen wiederherzustellen.

12. Set nach einem der Ansprüche 1 bis 11, **dadurch gekennzeichnet, dass** es mindestens eine sterile Filtervorrichtung aufweist, die mit einer Filtermembran versehen ist, deren Porengröße von 40 µm bis 200 µm beträgt.

13. Set nach Anspruch 12, **dadurch gekennzeichnet, dass** die sterile Filtervorrichtung ferner eine sterile Nadel von mindestens 16 Gauge aufweist.

14. Set nach einem der Ansprüche 1 bis 13, **dadurch gekennzeichnet, dass** es ferner mindestens eine Nadel von höchstens 18 Gauge aufweist.

15. Set nach einem der Ansprüche 1 bis 14, **dadurch gekennzeichnet, dass** es ferner eine Vorrichtung (2) aufweist, welche die Gewinnung einer Zusammensetzung aus einer Mischung von Vorläuferzellen und einer biologischen Matrix ermöglicht, wobei die Vorrichtung besteht aus:
(i) einer ersten Spritze (21), die zum Befüllen mit einer geeigneten Menge einer biologischen Matrix vorgesehen ist;
(ii) einer zweiten Spritze (22), die zum Befüllen mit einer Vorläuferzellensuspension in einem isotonischen Medium vorgesehen ist; und
(iii) einer Leitung (23), welche die Auslassöffnung jeder der Spritzen (21, 22) miteinander verbindet, wobei die Leitung (23) die Spritzen (21) und (22) in Fluidverbindung miteinander bringt.

16. Verfahren zur Gewinnung einer Fettzellen enthaltenden Zusammensetzung mit den folgenden Schritten:
a) Einbringen einer von einer Fettabsaugung oder Lipektomie stammenden Fettgewebesuspension in ein Rohr (1) nach Anspruch 1;
b) Durchführen einer enzymatischen Verdauung der Fettgewebesuspension in dem Rohr (1) mittels einer mindestens eine Protease enthaltenden enzymatischen Zubereitung;
c) Zentrifugieren der Suspension nach der enzymatischen Verdauung;
d) Sammeln der Fraktionen der zentrifugierten Suspension, welche die interessierenden Zellen enthalten:
d1) Sammeln der Fraktion (PA) der Vorläuferfettzellen, welche sich als Ablagerung am Boden des Röhrchens (1) befinden;
d2) gegebenenfalls Sammeln der Fraktion der reifen Fettzellen (AM), die sich in dem oberen Bereich der zentrifugierten Fettzellensuspension befinden;
e) Inkubieren der Fraktion (PA) in einer hypotonischen Lösung, um eine Lyse der Erythrozyten durchzuführen; und
f) Bilden einer Fettzellenzusammensetzung aus einer der folgenden Zellfraktionen:
f1) der am Ende des Schrittes e) erhaltenen Fraktion (PA) der Vorläuferfettzellen;
f2) der am Ende des Schrittes d1) erhaltenen Fraktion (AM) der reifen Fettzellen; und
f3) einer Mischung von Zellen der Fraktion (PA) und von Zellen der Fraktion (AM).

17. Verfahren nach Anspruch 16, **dadurch gekennzeichnet, dass** im Schritt f), wenn die Mischung gleichzeitig aus Zellen der Fraktion (PA) und aus Zellen der Fraktion (AM) gebildet wird, das Zellverhältnis (PA)/(AM) zwischen 1/99 und 99/1 variiert.

18. Zentrifugierbares steriles Röhrchen (1) mit einem Körper (11) und einem Stopfen (12), der ein Element aus elastischem und zurückziehbarem Material aufweist, wobei der Körper (11) durch den Stopfen (12) hermetisch geschlossen ist, wobei der Stopfen (12) eine Luftauslassöffnung (13) aufweist, welche mit einer Filtermembran mit einer Porengröße von weniger als 0,30 µm versehen ist.

19. Steriles Röhrchen (1) nach Anspruch 18, **dadurch gekennzeichnet, dass** der Stopfen (12) des sterilen Röhrchens (1) mindestens eine sterile Perforationsfläche (14) aufweist, die mit einer lösbaren Folie (15) abgedeckt ist.

20. Steriles Röhrchen (1) nach einem der Ansprüche 18 und 19, **dadurch gekennzeichnet, dass** der Stopfen (12) des sterilen Röhrchens mindestens zwei Perforationsflächen (14) aufweist.

21. Steriles Röhrchen (1) nach einem der Ansprüche 18 bis 20, **dadurch gekennzeichnet, dass** es eine für die Verdauung des Fettgewebes geeignete enzymatische Zubereitung enthält, wobei die enzymatische Zubereitung in flüssiger oder lyophilisierter Form vorliegt.
